(19) Europäisches Patentamt / European Patent Office / Office européen des brevets

(11) **EP 2 848 934 B1**

(12) **EUROPÄISCHE PATENTSCHRIFT**

(45) Veröffentlichungstag und Bekanntmachung des Hinweises auf die Patenterteilung:
**31.08.2022 Patentblatt 2022/35**

(21) Anmeldenummer: **13004491.0**

(22) Anmeldetag: **13.09.2013**

(51) Internationale Patentklassifikation (IPC):
**G01N 33/22** *(2006.01)* **G01N 25/18** *(2006.01)*

(52) Gemeinsame Patentklassifikation (CPC):
**G01N 33/225; G01N 25/18**

(54) **Verfahren und Sensor zur Bestimmung von Brennstoffeigenschaften von Gasgemischen**

Method and sensor for determining fuel characteristics of gas mixtures

Procédé et capteur pour la détermination des caractéristiques d'un mélange de carburants gazeux

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**18.03.2015 Patentblatt 2015/12**

(73) Patentinhaber: **MEMS AG**
**5200 BRUGG (CH)**

(72) Erfinder:
• **Prêtre, Philippe**
**5405 Dättwil (CH)**
• **Kempe, Andreas**
**8049 Zürich (CH)**

(74) Vertreter: **Steiner, Peter**
**Patente + Marken**
**Untere Wolfackerstrasse 16**
**8280 Kreuzlingen (CH)**

(56) Entgegenhaltungen:
**EP-A1- 0 439 950     EP-A2- 0 554 095**

**Beschreibung**

Die Erfindung bezieht sich auf ein Verfahren und auf einen Sensor zur Bestimmung einer ersten Materialeigenschaft von Gasgemischen. Unter Materialeigenschaften werden insbesondere brenntechnisch relevante Grössen wie der Energieinhalt des Gasgemisches, ausgedrückt durch den Brennwert oder den Wobbe-Index, die Methanzahl, die Dichte oder den Luftbedarf, verstanden.

Brennwert, Wobbe-Index, Methanzahl, Dichte und Luftbedarf sind wichtige Eigenschaften einer Gasmischung bei Gasanwendungen wie Gasfeuerungsregelungen, Motorregelungen bei Gasfahrzeugen, insbesondere Natural Gas Vehicles (NGV), und Gasblockheizkraftwerken, oder bei der Reformierung von Gas für Brennstoffzellen.

Das Verfahren und der Sensor gemäss vorliegender Erfindung können zur Steuerung von Verbrennungsprozessen (Gasfeuerungsregelung), zur Motorregelung von (Erd-)gasmotoren in Gasfahrzeugen und Gasblockheizkraftwerken oder zur Luftbedarfsbestimmung in Brennstoffzellen verwendet werden.

Eine erste Materialeigenschaft, beispielsweise die Dichte, soll oft bei einer bestimmten, durch einen Parameter charakterisierten Bedingung, beispielsweise bei einer bestimmten Temperatur T1, bestimmt werden, ist aber messtechnisch nicht oder nur mit grösserem Aufwand erfassbar. Eine bekannte Lösungsmöglichkeit sieht vor, dass eine Korrelation der ersten Materialeigenschaft, die nicht gemessen werden kann, mit einer zweiten Materialeigenschaft, die gemessen werden kann, beispielsweise der Wärmeleitfähigkeit, bei derselben Bedingung, zum Beispiel derselben Temperatur, benutzt wird. Die Korrelation kann beispielsweise in Form einer Wertetabelle oder einer Polynomnäherung in einem Datenspeicher des Sensors oder der Auswerteeinheit hinterlegt sein. Oft ist diese Korrelation aber nicht sehr gut.

Aus US 7188519 B2 ist eine Methode und ein Sensor zur Bestimmung eines Flusses und mindestens eines Parameter eines Fluids bekannt, wo die Messung bei zwei Temperaturen zur Bestimmung der Wärmeleitfähigkeit dient, jedoch das Resultat dieser Bestimmung nicht in Beziehung zu anderen Materialeigenschaften des Fluids gesetzt wird.

Die Bestimmung von Gaseigenschaften mittels der Messung der Wärmeleitfähigkeit λ des Gases bei zwei verschiedenen Temperaturen ist in der Literatur z.B. durch C. Rahmouni, O. Le Corre, M Tazerout im Artikel "Online determination of natural gas properties", C.R: Mécanique 331 (2003, pp. 545-550), beschrieben.

In EP 0 554 095 A2 wird eine Vorrichtung und ein Verfahren beschrieben, in welchen eine charakteristische Brennstoffeigenschaft bei Standardbedingungen aus Messungen der Wärmeleitfähigkeit und der Wärmekapazität bestimmt werden.

EP 0 439 950 A1 beschreibt ein Verfahren und eine Vorrichtung zur Bestimmung von Brennstoffeigenschaften bei Standardbedingungen unter Verwendung eines mikrothermischen Sensors.

Aus EP 2 015 056 B1 ist ein thermischer Durchflusssensor zur Bestimmung einer brenntechnisch relevanten Grösse bekannt, basierend auf einer Wärmeleitfähigkeitsmessung bei bekanntem Massenfluss. Die Zuordnung zwischen brenntechnisch relevanter Grösse und Wärmeleitfähigkeit ist jedoch bei realen Bedingungen (z.B. Raumtemperatur) nicht für alle Gasgemische eineindeutig, weshalb der Sensor nicht zwischen H- und L-Gasen unterscheiden kann.

Der Erfindung liegt daher die Aufgabe zugrunde, ein Verfahren zur Bestimmung einer ersten Materialeigenschaft, insbesondere eines Gasgemisches mittels Korrelation zwischen der ersten Materialeigenschaft und einer zweiten Materialeigenschaft anzugeben, wobei in dem Verfahren die Korrelation zwischen der ersten und der zweiten Materialeigenschaft verbessert wird. Des Weiteren wird ein Sensor mit einer Auswerteeinheit angegeben, die nach diesem Verfahren arbeitet.

Die Aufgabe wird bezüglich des Verfahrens durch ein Verfahren nach Anspruch 1 gelöst.

Bezüglich des Sensors wird die Aufgabe durch einen Sensor nach Anspruch 9 gelöst.

In dem erfindungsgemässen Verfahren zur Bestimmung einer ersten Materialeigenschaft, insbesondere einer brenntechnisch relevanten Grösse eines Gasgemisches, bei einer ersten, von einem Parameter charakterisierten Bedingung, zum Beispiel einer ersten Temperatur, wird eine zweite Materialeigenschaft erfasst und die erste Materialeigenschaft mittels einer Korrelation zwischen der ersten Materialeigenschaft und der zweiten Materialeigenschaft bestimmt. Das Verfahren zeichnet sich dadurch aus, dass in der genannten Korrelation jeweils Werte der ersten Materialeigenschaft, gegeben bei der ersten Bedingung, mit Werten der zweiten Materialeigenschaft, betrachtet bei einer zweiten, veränderten Bedingung, zum Beispiel einer zweiten Temperatur, korreliert werden. Um auf die Werte der zweiten Materialeigenschaft bei der veränderten Bedingung zu kommen, bedarf es einer Transformation, die aus der Abhängigkeit der zweiten Materialeigenschaft von dem die Bedingungen charakterisierenden Parameter hervorgeht.

Mit anderen Worten wird die Aufgabe bezüglich des Verfahrens z.B. gelöst durch ein Verfahren zur Bestimmung von Materialeigenschaften eines Gasgemisches, insbesondere brenntechnisch relevanter Grössen des Gasgemisches, ausgedrückt durch den Brennwert, den Wobbe-Index, die Methanzahl oder den Luftbedarf, mittels einer Korrelation zwischen den Materialeigenschaften und der Wärmeleitfähigkeit λ dieses Gasgemisches. Diese Korrelation ist im Allgemeinen nicht besonders gut, wenn sowohl die Materialeigenschaft als auch die Wärmeleitfähigkeit bei den gleichen Bedingungen, zum Beispiel bei Raumtemperatur, betrachtet werden. Erst wenn die Werte der Wärmeleitfähigkeit bei veränderten, (messtechnisch oft) nicht zugänglichen Temperaturen zur Korrelation mit der immer noch bei Raumtemperatur betrachteten Materialeigenschaft gebracht werden, ist es möglich, die Korrelation erheblich zu verbessern. Die Wärmeleitfähigkeit λ wird bei mindestens zwei Temperaturen gemessen, die zum Beispiel im Bereich von 0°C und 40°C liegen können, um durch eine Transformation, beispielsweise durch lineare Extrapolation, der gemessenen Werte in den für eine verbesserte Korrelation notwendigen Temperaturbereich der Wärmeleitfähigkeit λ vorzustossen.

Gemäss einer vorteilhaften Ausführungsform der Erfindung ist der die Bedingungen charakterisierende Parameter die Temperatur und/oder fallweise der Druck. Die erste Bedingung kann beispielsweise durch die Normalbedingungen, d.h. eine Temperatur von 0 °C und einen Druck von 1013.25 mbar, oder durch die Raumtemperatur von typisch 20 °C oder eine andere Temperatur im Bereich von 0 °C bis 40 °C gegeben sein, und die zweite Bedingung beispielsweise durch eine Temperatur ausserhalb des Bereichs von 0 °C bis 40 °C oder -10 °C bis 50 °C, zum Beispiel durch eine Temperatur zwischen 70 °C und 140 °C.

Unabhängig davon kann die Transformation beispielsweise durch einen linearen Zusammenhang oder einen Polynomausdruck oder eine Potenzreihe oder einen funktionalen Zusammenhang gegeben sein.

Die Werte der zweiten Materialeigenschaft bei der veränderten Bedingung können beispielsweise durch eine Transformation der Werte, erfasst bei der ersten Bedingung, erhalten werden.

Gemäss einer weiteren vorteilhaften Ausführungsform der Erfindung wird die Transformation der Werte der zweiten Materialeigenschaft mittels mindestens eines Messpunkts, erfasst bei der ersten Bedingung, etabliert.

Gemäss einer weiteren vorteilhaften Ausführungsform der Erfindung ist die zweite Materialeigenschaft die Wärmeleitfähigkeit λ eines Gasgemisches.

Gemäss einer anderen vorteilhaften Ausführungsform der Erfindung ist die erste Materialeigenschaft die Dichte oder die Wärmekapazität oder die Wärmediffusivität (Wärmeleitfähigkeit dividiert durch Dichte und Wärmekapazität) oder die Methanzahl oder der Wobbe-Index oder der Brennwert oder der Luftbedarf eines Gasgemisches.

Gemäss einer weiteren vorteilhaften Ausführungsform der Erfindung wird die Wärmediffusivität zur H-/L-Gas-Unterscheidung verwendet.

Der erfindungsgemässe Sensor zur Bestimmung einer brenntechnisch relevanten Grösse eines Gasgemisches enthält eine Auswerteeinheit, die zur Ausführung eines Verfahrens gemäss einer der oben beschriebenen Ausführungsformen und -varianten eingerichtet ist.

In einer vorteilhaften Ausführungsform umfasst der Sensor zusätzlich einen Sensorblock, der beispielsweise zusammen mit der Auswerteeinheit in einer gemeinsamen Baueinheit zusammengefasst sein kann.

In einer weiteren vorteilhaften Ausführungsform des Sensors ist der Sensorblock mit einem Hitzdrahtanemometer, insbesondere einem integrierten CMOS-Hitzdrahtanemometer, ausgestattet, welches mit dem Gasgemisch beaufschlagt und mittels welchem die Abhängigkeit der Wärmeleitfähigkeit (λ) bezüglich der Temperatur durch Messen bei mindestens zwei Temperaturen bestimmt werden kann, zum Beispiel durch Variation der Heizleistung und/oder das Vorhandensein zweier Temperaturmessstellen in wärmeleittechnisch unterschiedlichen Abständen zum Heizelement.

In anderen Worten wird die Aufgabe bezüglich des Sensors beispielsweise gelöst durch einen Sensor zur Bestimmung von Materialeigenschaften eines Gasgemisches mit einem Sensorblock mit Hitzdrahtanemometer, welches mit dem Gasgemisch beaufschlagt werden kann, und bei welchem die vom Heizelement ausgehende Wärme an mindestens einer Stelle mittels mindestens eines Temperatursensors gemessen wird, wobei entweder die Heizleistung variiert werden kann und/oder mehrere Temperatursensoren sich in wärmeleittechnisch unterschiedlichen Abständen zum Heizelement befinden. In wärmeleittechnisch unterschiedlichen Abständen bedeutet in diesem Zusammenhang, dass ein unterschiedlicher Wärmefluss ausgehend vom Heizelement hin zur Messstelle erzeugt werden kann, bedingt durch ungleiche, geometrische Verhältnisse und/oder unterschiedliches Wärmeleitvermögen der beteiligten Materialien.

Weiter umfasst die Erfindung eine Sensoranordnung mit einem Sensor gemäss einer der oben beschriebenen Ausführungsformen, wobei der Sensorblock mit Hitzdrahtanemometer an seinem Gas-Einlass an eine zu einer Gashauptleitung führende Messleitung angeschlossen und an seinem Gas-Auslass mit einem Absperrventil verbunden ist, das während der Messung geschlossen und zur Spülung des Sensors geöffnet werden kann.

Gemäss einer anderen vorteilhaften Ausführungsform der Sensoranordnung ist der Sensorblock mit Hitzdrahtanemometer direkt in der Gashauptleitung montiert.

Gemäss einer weiteren vorteilhaften Ausführungsform der Sensoranordnung ist der Sensorblock mit Hitzdrahtanemometer an seinem Gas-Einlass und Gas-Auslass an eine zu einer Gashauptleitung führende Messleitung angeschlossen (Bypassausführung).

Weiter gehört zur Erfindung die Verwendung eines Sensors nach einer der oben beschriebenen Ausführungsformen oder einer Sensoranordnung nach einer der oben beschriebenen Ausführungsformen zur Bestimmung und/oder Ausgabe der Gasart H- und/oder L-Gas, damit sich Gasverbraucher und Gasmessgeräte automatisch auf H- oder L-Gas einstellen können.

Die mit der Erfindung erzielbaren Vorteile bestehen darin, dass die Korrelation zwischen einer ersten, nicht oder nur mit grösserem Aufwand messbaren Materialeigenschaft und einer zweiten, messbaren Materialeigenschaft verbessert werden kann, und dass aufgrund der kostengünstigen Herstellbarkeit des nach dem erfindungsgemässen Verfahren arbeitenden Sensors mit Hitzdrahtanemometer Massenmarktanwendungen möglich sind. Es ist eine vergleichsweise hohe Messgenauigkeit erzielbar, die für die Dichte im Bereich von ± 1% liegt.

Weitere Vorteile sind aus der nachstehenden Beschreibung ersichtlich.

Vorteilhafte Ausgestaltungen der Erfindung sind in den Unteransprüchen gekennzeichnet.

Die Erfindung wird nachstehend anhand der in der Zeichnung dargestellten Ausführungsbeispiele erläutert. Es zeigen:

Fig. 1    Schema des der Erfindung zugrunde liegende Transformationsprinzips,

Fig. 2, 3, 4    Ausführungsbeispiele für den schematischen Aufbau einer Sensoranordnung zur Bestimmung von Materialeigenschaften, insbesondere von brennbaren Gasen, gemäss vorliegender Erfindung,

Fig. 5a-c    ein Beispiel der Korrelation zwischen der Dichte und der Wärmeleitfähigkeit $\lambda$ unterschiedlicher Gase bei drei verschiedenen Temperaturen,

Fig. 6    ein Beispiel einer Temperaturtransformation der Wärmeleitfähigkeit $\lambda$ für zwei verschiedene Gasgemische vom Temperaturbereich T1 und T2 in den Bereich T3.

Das erfindungsgemässe Verfahren umfasst also die Bestimmung von Materialeigenschaften, z.B. Brennwert, Wobbe-Index, Methanzahl, Dichte oder Luftbedarf eines Gasgemisches basierend auf einer mikrothermischen Bestimmung der Wärmeleitfähigkeit des Gasgemisches. Ausgegangen wird dabei von der das System beschreibenden, eindimensionalen Wärmeleitungsgleichung

$$\nabla^2 T = -\frac{1}{\lambda}\Theta, \qquad (1)$$

wobei

T    die Temperatur,

$\lambda$    die Wärmeleitfähigkeit des Gasgemisches,

$\nabla^2 T$    der Laplace-Operator, angewandt auf die Temperatur T bedeuten,

wobei

$$\nabla^2 = \left(\frac{d}{dx}\right)^2 + \left(\frac{d}{dy}\right)^2 + \left(\frac{d}{dz}\right)^2.$$

$\Theta$ mit der Einheit Watt/m³ beschreibt den Quellterm des Heizelements eines Hitzdrahtanemometers, das Wärmeenergie in das System speist. Aus Gleichung (1) wird ersichtlich, dass die Wärmeleitfähigkeit $\lambda$ wegen des Quellterms $\Theta$ auf die Lösung der Gleichung (1) einwirkt. Kann demnach die Heizleistung verändert und/oder die Temperatur an wärmeleittechnisch unterschiedlichen Punkten gemessen werden, lässt sich auf die Wärmeleitfähigkeit $\lambda$ des Gases bei verschiedenen Temperaturen schliessen.

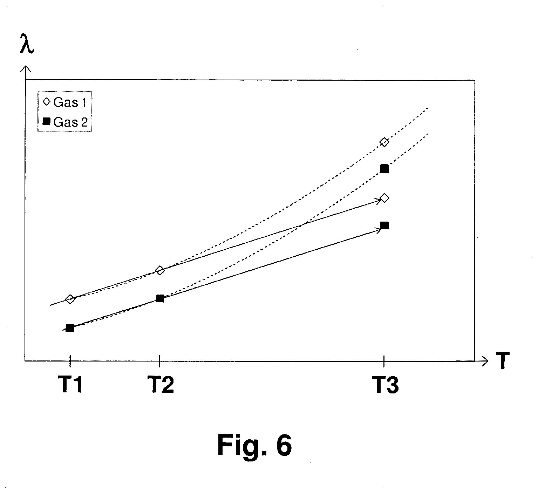

In dem Verfahren gemäss vorliegender Erfindung werden jeweils Werte der ersten Materialeigenschaft $M_1$, p, bestimmt bei der ersten Bedingung $p_1$, $T_1$, mit Werten der zweiten Materialeigenschaft $M_2$, $\lambda$, betrachtet bei der zweiten Bedingung $p_3$, $T_3$, korreliert. Betreffend die Werte der zweiten Materialeigenschaft bei der zweiten Bedingung $p_3$, $T_3$ siehe z.B. Fig. 6.

Mit Kenntnis der Wärmeleitfähigkeit $\lambda$ des Gases bei verschiedenen Temperaturen lässt sich die eingangs beschriebene Temperaturtransformation der Wärmeleitfähigkeit $\lambda$ durchführen und eine verbesserte Korrelation zu brenntechnisch relevanten Grössen herstellen.

Von den mathematischen Methoden der Physik sind mehrere Verfahren bekannt, die es erlauben, komplizierte mathematische Zusammenhänge mittels Transformationen in einen anderen mathematischen Raum einfach darzustellen. Als Beispiel sei die Laplace-Transformation genannt, bei der die Differentiation auf eine Multiplikation zurückgeführt werden kann, was das Lösen von Differentialgleichungen erheblich erleichtert. Durch eine Rück-Transformation erhält man dabei eine Lösung des ursprünglichen Problems.

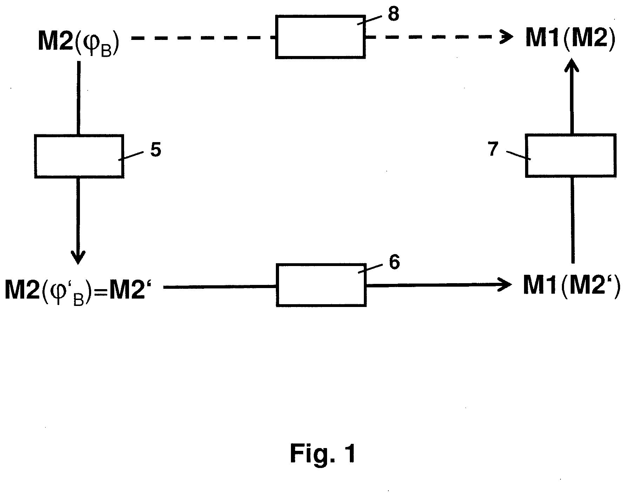

Wie Fig. 1 zeigt, macht die Erfindung von dem Prinzip der Transformation Gebrauch beim Bestimmen einer ersten Materialeigenschaft M1, beispielsweise der Dichte, die messtechnisch nicht zugänglich ist, mittels Korrelation der ersten Materialeigenschaft mit einer zweiten Materialeigenschaft M2, beispielsweise der Wärmeleitfähigkeit, die messtechnisch zugänglich ist, indem sie die zweite Materialeigenschaft M2 bei soweit veränderten Bedingungen betrachtet, bis ein verbesserter Zusammenhang, in anderen Worten eine verbesserte Korrelation 6, zwischen den Materialeigenschaften M1 und M2' entsteht. Die Änderung der zweiten Materialeigenschaft M2 unter veränderten Bedingungen geht aus deren Abhängigkeit von dem die Bedingungen charakterisierenden Parameters $\varphi_B$, beispielsweise der Temperatur, hervor und ist durch eine Transformation 5 der Werte der zweiten Materialeigenschaft M2 ($\varphi_B$) gegeben, die unter gleichen, unveränderten Bedingungen bezüglich des Parameters $\varphi_B$ bestimmt werden kann, für die die erste Materialeigenschaft M1 bestimmt werden soll. Wird nun die zweite, transformierte Materialeigenschaft M2' = M2 ($\varphi_B$') in Beziehung zur ersten, nicht transformierten Materialeigenschaft M1 gesetzt, kann durch die auf verschiedene Materialien unterschiedlich wirkende Transformation 5 eine verbesserte Beziehung, d.h. Korrelation 6, zwischen den Materialeigenschaften M1 und M2' der verschiedenen Materialien hergestellt werden. Diese Beziehung zur ersten Materialeigenschaft M1 wäre unter den ursprünglichen Bedingungen $\varphi_B$, d.h. in der ursprünglichen Korrelation 8, nicht auf gleich gute Art möglich gewesen. Durch anschliessendes Zuordnen der so erhaltenen Werte für die Materialeigenschaft M1 zu den Werten der Materialeigenschaft $M2(\varphi_B)$ bei der ursprünglichen Bedingung $\varphi_B$ wird der Zusammenhang zwischen den Materialeigenschaften M1 und M2 unter physikalisch realen Bedingungen wieder hergestellt. Dieser letzte Schritt entspricht der in Fig. 1 gezeigten Rück-Transformation 7.

In Bezug auf das Verfahren und auf den in der Erfindung genannten Sensor zur Bestimmung einer ersten Materialeigenschaft, insbesondere von Gasgemischen, verhindert die von Gasgemisch zu Gasgemisch sehr unterschiedlich in Erscheinung tretende Abhängigkeit dieser ersten Materialeigenschaft und einer zweiten Materialeigenschaft von beispielsweise der Temperatur T als den die Bedingungen charakterisierender Parameter $\varphi_B$ ein-eindeutige Zusammenhänge zwischen der ersten und zweiten Materialeigenschaft im normalerweise zugänglichen Temperaturbereich (z.B. bei Raumtemperatur).

Die Transformation 5 kann über die Abhängigkeit der zweiten Materialeigenschaft M2 von dem die Bedingungen charakterisierenden Parameter $\varphi_B$ selbst erhalten werden. Ändert die zweite Materialeigenschaft M2 dabei ihre Grösse z.B. als Funktion der Temperatur T, lässt sich mit Kenntnis dieser Funktion die Transformation in den anderen Temperaturbereich durchführen. Dabei ist es unerheblich, ob diese Funktion eine physikalisch korrekte ist oder nicht. Solange sie im nicht transformierten Bereich die Materialeigenschaft M2 mit genügender Genauigkeit abbildet und die betrachtete Materialgruppe fundamental das gleiche gesetzmässige Verhalten in Bezug auf den Parameters $\varphi_B$ zeigt, müssen die transformierten Grössen nicht zwingend die realen Werte abbilden.

Das Verfahren lässt sich selbst dann anwenden, wenn die Transformation der zweiten Materialeigenschaft M2 in Bezug auf den Parameters $\varphi_B$ in unsinnige Bereiche führt, z.B. in den Temperaturbereich T < 0 Kelvin, da die zu bestimmende, erste Materialeigenschaft M1 dieser Transformation nicht unterzogen wird.

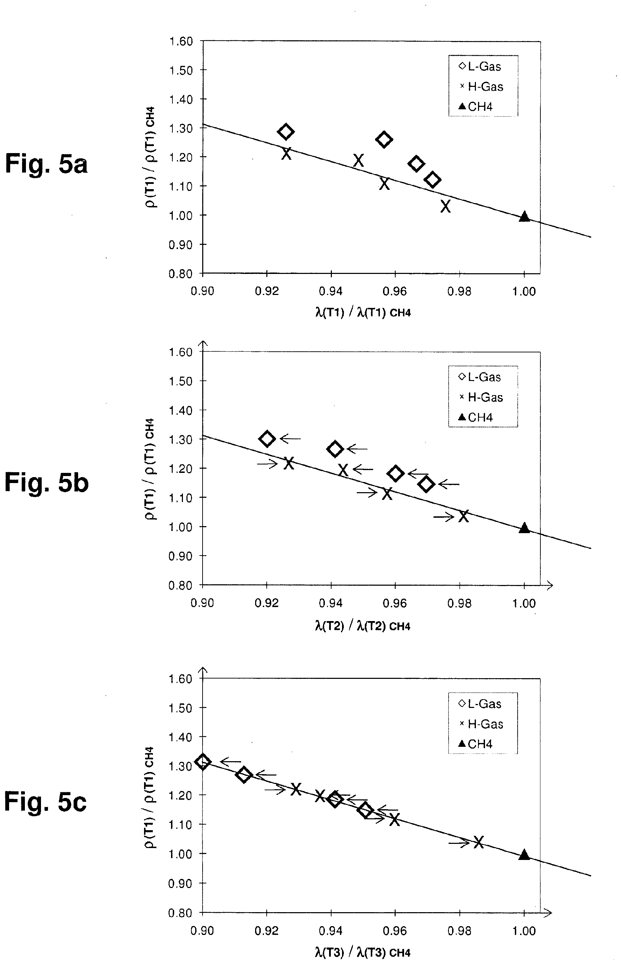

In Fig. 5a-c ist beispielhaft eine Korrelation zwischen der Dichte $\rho / \rho_{CH4}$ (Ordinate, y-Achse) und der Wärmeleitfähigkeit $\lambda / \lambda_{CH4}$ (Abszisse, x-Wert) verschiedener Gasgemische in Bezug auf Methan ($CH_4$) dargestellt. Die Figuren 5a - 5c zeigen jeweils den Kurvenverlauf für Abszissenwerte bei drei unterschiedlichen Temperaturen T1, T2 und T3, wobei T1 < T2 < T3 gilt. Für die Ordinatenwerte bleibt die Temperatur T = T1 in den drei Figuren unverändert.

Wie Fig. 5c zeigt, besteht zwischen der Dichte $\rho / \rho_{CH4}$ bei der Temperatur T1 und der Wärmeleitfähigkeit $\lambda / \lambda_{CH4}$ bei der Temperatur T3 eine hinreichend gute Korrelation, wobei sich diese im Vergleich zu den niedrigeren Temperaturen T1 und T2 deutlich verbessert hat (siehe Figuren 5a und 5b). Da die Dichte immer bezüglich der Temperatur T1 betrachtet wird, werden die Datenpunkte der verschiedenen Gasgemische durch die Temperaturtransformation der Wärmeleitfähigkeit parallel zur Abszisse in unterschiedlichem Masse verschoben, sodass sie, ausgehend von der zerstreut angeordneten Punkteschar in Fig. 5a (entsprechend einer vergleichsweise schlechten Korrelation), in Fig. 5c annähernd auf eine Linie zu liegen kommen (entsprechend einer verbesserten Korrelation).

In ähnlicher Weise erlaubt das Verfahren und der Sensor die Unterscheidung der Erdgase in H-Gase ("High Calorific Value", z. B. Erdgas aus Russland und der Nordsee) und L-Gase ("Low Calorific Value", z. B. Erdgas aus Holland) (definitionsgemäss bei einem Brennwert von ca. 10 kWh/m$^3$ bzw. einem Wobbe-Index von ca. 13 kWh/m$^3$ für eine Temperatur von 0°C und einen Druck von 1013.25 mbar), wenn die Wärmediffusivität, d.h. Wärmeleitfähigkeit dividiert durch Dichte und Wärmekapazität, betrachtet wird. Bezüglich der Korrelation zur Wärmeleitfähigkeit $\lambda$ muss bei der Temperatur T3 nicht zwischen H- bzw. L-Gasen unterschieden werden, bei den Temperaturen T1 bzw. T2 hingegen schon, wobei T1 < T2 < T3 gilt. Als eine weitere Anwendung, nach erfolgter Korrelation im Temperaturbereich T3, erlaubt die Rück-Transformation in den Bereich von T1 und T2 eine Unterscheidung zwischen H- und L-Gasen, was für Gasverbraucher und Gasmessgeräte die automatische Einstellung auf H- oder L-Gas ermöglicht.

In Fig. 6 ist ein Beispiel einer möglichen Temperaturtransformation der Wärmeleitfähigkeit $\lambda$ vom Temperaturbereich T1 und T2 in den Bereich T3 für zwei verschiedene Gasgemische Gas 1 und Gas 2 dargestellt, wobei die physikalische Realität (gestrichelte Linien) nicht unbedingt korrekt wiedergegeben werden muss (ausgezogene Linien), solange im Ausgangsbereich zwischen T1 bzw. T2 die physikalischen Werte durch die Transformation korrekt wiedergegeben werden.

Auch wenn mit dem Sensorelement des Hitzdrahtanemometers dank der Möglichkeit der variierenden Heizleistung und/oder dem Vorhandensein mehrerer, wärmeleittechnisch unterschiedlich vom Heizelement entfernt gelegener Temperatursensoren inhärent die Wärmeleitfähigkeit $\lambda$ bei den Temperaturen T1 und T2 gemessen werden kann, lässt sich die Wärmeleitfähigkeit $\lambda$ bei der Temperatur T3 nicht ohne Zusatzaufwand bestimmen, z.B. durch aufwändige Temperierung des Sensorelements.

Trotzdem ist es mit der in Fig. 6 gezeigten Temperaturtransformation möglich, die Wärmeleitfähigkeit $\lambda$ im sonst nur schwer zugänglichen Temperaturbereich zu bestimmen und die in Fig. 5c gezeigte, verbesserte Korrelation zur Bestimmung der Dichte auszunützen. Dabei ist es nicht relevant, ob die benützte Transformation die physikalischen Gegebenheiten bei der Temperatur T3 tatsächlich richtig wiedergibt, sondern nur, ob die Transformation, ausgehend von der korrekten Beschreibung der physikalischen Gegebenheiten bei den Temperaturen T1 und T2, derart gewählt wird, dass sich eine verbesserte Korrelation bei der Temperatur T3 ergibt, und dass die betrachtete Materialgruppe fundamental das gleiche gesetzmässige Verhalten bezüglich der Temperatur zeigt. So stimmt die als Transformation beispielhaft gezeigte lineare Extrapolation der Werte im Bereich T1 bzw. T2 gut mit der physikalisch korrekten, quadratischen Abhängigkeit überein, im transformierten Bereich T3 jedoch nicht mehr. Dies spielt bezüglich des Verfahrens aber keine Rolle, da viele Gase zwar fundamental ein quadratisches Verhalten bezüglich der Temperatur zeigen, aber selbst bei linearer Extrapolation sich relativ ähnliche Verhältnisse zwischen den Gasen ergeben.

In einer vorteilhaften Ausführungsform basiert die mikrothermische Wärmeleitfähigkeitsmessung auf einem integrierten CMOS-Hitzdrahtanemometer. Zu dieser Technologie wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 hingewiesen.

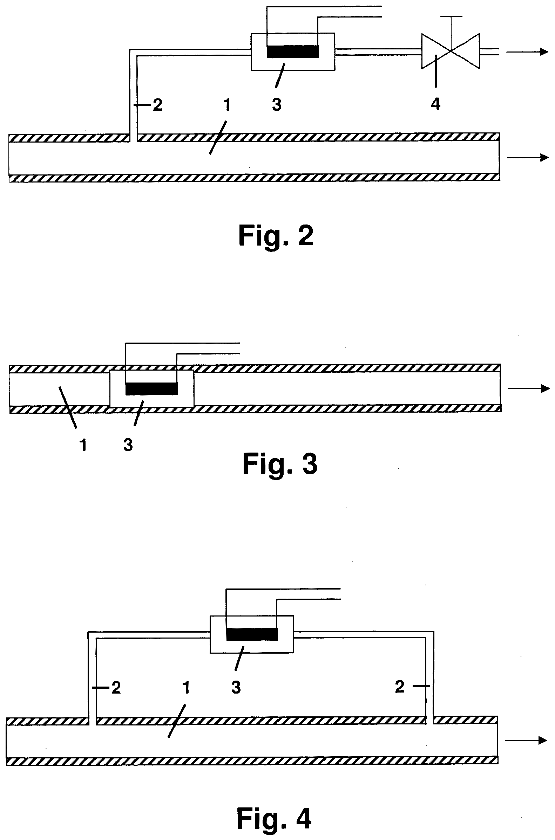

Ein einfacher Aufbau eines Sensors zur Messung brenntechnisch relevanter Grössen von Gasen ist in Fig. 2 gezeigt. Gas aus der Gashauptleitung 1, welche z.B. die Zufuhrleitung zum Motor eines NGV ist, wird über eine Messleitung 2 zum mikrothermischen Sensor 3 (Sensorblock mit Hitzdrahtanemometer) geführt. Im Sensor 3 werden die Wärmeleitfähigkeit $\lambda$ und die Temperatur T bestimmt und über die Temperaturtransformation die Korrelation mit der gesuchten Materialeigenschaft hergestellt. Ein Absperrventil 4 am Auslass des Sensors 3 dient dazu, den Gasfluss zu unterbrechen bzw. sich in Intervallen zur Spülung der Messleitung zu öffnen. Alternativ kann der mikrothermische Sensor 3 (Sensorblock mit Hitzdrahtanemometer) direkt in die Gashauptleitung 1 integriert werden (Fig. 3), wo ein direkter Gasaustausch stattfindet. Alternativ kann der mikrothermische Sensor 3 (Sensorblock mit Hitzdrahtanemometer) in einem Bypass 2 zur Gashauptleitung 1 integriert werden (Fig. 4), wo der Gasaustausch über Diffusion stattfindet.

Das oben beschriebene Verfahren und der oben beschriebene Sensor zur Bestimmung einer ersten Materialeigenschaft mittels Korrelation mit einer zweiten, durch Messung erfassten Materialeigenschaft haben den Vorteil, dass die Korrelation zwischen der ersten Materialeigenschaft und der zweiten Materialeigenschaft zur Verbesserung der Genauigkeit angepasst werden kann, indem die erste Materialeigenschaft bei einer ersten Bedingung mit der zweiten Materialeigenschaft, betrachtet bei einer zweiten, veränderten Bedingung, korreliert wird, wobei die zweite Bedingung nicht der Bedingung bei der Messung zu entsprechen braucht, sondern dank der Transformation der Messwerte abweichend von der oder den Messbedingungen gewählt werden kann. Damit kann die Korrelation auf wirtschaftliche Weise verbessert werden, selbst wenn die zweite Bedingung messtechnisch nicht oder nur mit vergleichsweise grossem Aufwand zugänglich ist.

## Patentansprüche

1. Verfahren zur Bestimmung einer ersten Materialeigenschaft ($M_1$, $\rho$) eines Gasgemisches, insbesondere einer brenntechnisch relevanten Grösse eines Gasgemisches, bei einer ersten, von einem Parameter (p, T) charakterisierten Bedingung ($p_1$, $T_1$), zum Beispiel einer ersten Temperatur, wobei in dem Verfahren eine zweite Materialeigenschaft ($M_2$, $\lambda$) erfasst und die erste Materialeigenschaft mittels einer Korrelation ($M_1(M_2)$, $\rho(\lambda)$) zwischen der ersten Materialeigenschaft und der zweiten Materialeigenschaft bestimmt wird, **dadurch gekennzeichnet, dass** in der genannten Korrelation ($M_1(M_2)$, $\rho(\lambda)$) jeweils Werte der ersten Materialeigenschaft ($M_1$, $\rho$), gegeben bei der ersten Bedingung ($p_1$, $T_1$), mit Werten der zweiten Materialeigenschaft ($M_2$, $\lambda$), betrachtet bei einer zweiten, veränderten Bedingung ($p_3$, $T_3$), zum Beispiel einer zweiten Temperatur, korreliert werden, wobei die Werte der zweiten Materialeigenschaft bei der veränderten Bedingung durch eine Transformation erhalten werden und die Transformation aus der Abhängigkeit der zweiten Materialeigenschaft von dem die Bedingungen charakterisierenden Parameter (p, T) hervorgeht.

2. Verfahren nach Anspruch 1, wobei der die Bedingungen charakterisierende Parameter die Temperatur und/oder der Druck ist.

3. Verfahren nach Anspruch 1 oder 2, wobei die Transformation durch einen linearen Zusammenhang oder einen Polynomausdruck oder eine Potenzreihe oder einen funktionalen Zusammenhang gegeben ist.

4. Verfahren nach einem der Ansprüche 1 bis 3, wobei die Werte der zweiten Materialeigenschaft bei der veränderten Bedingung durch eine Transformation der Werte, erfasst bei der ersten Bedingung ($p_1$, $T_1$), erhalten werden.

5. Verfahren nach einem der Ansprüche 1 bis 4, **dadurch gekennzeichnet, dass** die Transformation der Werte der zweiten Materialeigenschaft ($M_2$, $\lambda$) mittels mindestens eines Messpunkts, erfasst bei der ersten Bedingung ($p_1$, $T_1$), etabliert wird.

6. Verfahren nach einem der Ansprüche 1 bis 5, **dadurch gekennzeichnet, dass** die zweite Materialeigenschaft die Wärmeleitfähigkeit ($\lambda$) eines Gasgemisches ist.

7. Verfahren nach einem der Ansprüche 1 bis 6, **da-**

**durch gekennzeichnet, dass** die erste Materialeigenschaft die Dichte (ρ) oder die Wärmekapazität (c_p) oder die Wärmediffusivität (Wärmeleitfähigkeit dividiert durch Dichte und Wärmekapazität) oder die Methanzahl oder der Wobbe-Index oder der Brennwert oder der Luftbedarf eines Gasgemisches ist.

8. Verfahren nach Anspruch 7, wobei die Wärmediffusivität zur H-/L-Gas-Unterscheidung verwendet wird.

9. Sensor zur Bestimmung einer brenntechnisch relevanten Grösse eines Gasgemisches mit einer Auswerteeinheit, die zur Ausführung eines Verfahrens gemäss einem der Ansprüche 1 bis 8 eingerichtet ist.

10. Sensor nach Anspruch 9 umfassend zusätzlich einen Sensorblock (3), insbesondere einen Sensorblock (3), der zusammen mit der Auswerteeinheit in einer gemeinsamen Baueinheit zusammengefasst ist.

11. Sensor nach Anspruch 10, wobei der Sensorblock (3) mit einem Hitzdrahtanemometer, insbesondere einem integrierten CMOS-Hitzdrahtanemometer, ausgestattet ist, welches mit dem Gasgemisch beaufschlagt und mittels welchem die Abhängigkeit der Wärmeleitfähigkeit (λ) bezüglich der Temperatur durch Messen bei mindestens zwei Temperaturen bestimmt werden kann, zum Beispiel durch Variation der Heizleistung und/oder das Vorhandensein zweier Temperaturmessstellen in wärmeleittechnisch unterschiedlichen Abständen zum Heizelement.

12. Sensoranordnung mit einem Sensor nach Anspruch 10 oder 11, wobei der Sensorblock (3) an seinem Gas-Einlass an eine zu einer Gashauptleitung (1) führende Messleitung (2) angeschlossen und an seinem Gas-Auslass mit einem Absperrventil (4) verbunden ist, das während der Messung geschlossen und zur Spülung des Sensors geöffnet werden kann.

13. Sensoranordnung mit einem Sensor nach Anspruch 10 oder 11, wobei der Sensorblock (3) in der Gashauptleitung (1) montiert ist.

14. Sensoranordnung mit einem Sensor nach Anspruch 10 oder 11, wobei der Sensorblock (3) an seinem Gas-Einlass und Gas-Auslass an eine zu einer Gashauptleitung (1) führende Messleitung (2) (Bypass) angeschlossen ist.

**Claims**

1. Method for determining a first material property ($M_1$, ρ) of a gas mixture, in particular a combustion-related variable of a gas mixture, at a first condition ($p_1$, $T_1$) **characterized by** a parameter, for example a first temperature, in which method a second material property ($M_2$, λ) is detected and the first material property is determined by means of a correlation ($M_1(M_2)$, ρ(λ)) between the first material property and the second material property, **characterized in that** in said correlation ($M_1(M_2)$, ρ(λ)) values of the first material property ($M_1$, ρ), given at the first condition ($p_1$, $T_1$), are correlated respectively with values of the second material property ($M_2$, λ), considered at a second, modified condition ($p_3$, $T_3$), for example a second temperature, the values of the second material property being obtained at the changed condition by a transformation and the transformation resulting from the dependency of the second material property from the parameter (p, T) characterizing the conditions.

2. Method according to claim 1, wherein the parameter characterizing the conditions is the temperature and/or the pressure.

3. Method according to claim 1 or 2, in which the transformation is given by a linear relationship or a polynomial expression or a power series or a functional relationship.

4. Method according to one of claims 1 to 3, wherein the values of the second material property at the modified condition are obtained by a transformation of the values detected at the first condition ($p_1$, $T_1$).

5. Method according to one of claims 1 to 4, **characterized in that** the transformation of the values of the second material property ($M_2$, λ) is set up by means of at least one measurement point detected at the first condition ($p_1$, $T_1$).

6. Method according to one of claims 1 to 5, **characterized in that** the second material property is the thermal conductivity (λ) of a gas mixture.

7. Method according to one of claims 1 to 6, **characterized in that** the first material property is the density (ρ) or the heat capacity ($c_p$) or the heat diffusivity (thermal conductivity divided by density and heat capacity) or the methane number or the Wobbe index or the calorific value or the air requirement of a gas mixture.

8. The method of claim 7, wherein thermal diffusivity is used for H-/L gas discrimination.

9. Sensor for determining a combustion-related variable of a gas mixture with an evaluation unit that is set up to carry out a method according to one of claims 1 to 8.

**10.** Sensor according to claim 9, additionally comprising a sensor block (3), in particular a sensor block (3), which is combined with the evaluation unit in a common structural unit.

**11.** Sensor according to claim 10, wherein the sensor block (3) is equipped with a hot-wire anemometer, in particular an integrated CMOS hot-wire anemometer, which can be exposed to the gas mixture and by means of which the dependency of the thermal conductivity ($\lambda$) with respect to the temperature can be determined by measuring at least at two temperatures, for example, by varying the heating power and/or by the presence of two temperature measuring points at different distances from the heating element in terms of heat conduction.

**12.** Sensor arrangement with a sensor according to claim 10 or 11, wherein the sensor block (3) is connected at its gas inlet to a measuring line (2) leading to a main gas line (1) and is connected at its gas outlet to a shutoff valve (4), which can be closed during the measurement and opened to flush the sensor.

**13.** Sensor arrangement with a sensor according to claim 10 or 11, wherein the sensor block (3) is mounted in the gas main line (1).

**14.** Sensor arrangement with a sensor according to claim 10 or 11, the sensor block (3) being connected at its gas inlet and gas outlet to a measuring line (2) (bypass) leading to a main gas line (1).

**Revendications**

**1.** Procédé pour déterminer une première propriété de matériau ($M_1$, $\rho$) d'un mélange gazeux, en particulier une grandeur d'un mélange gazeux pertinente pour la technique de combustion, dans une première condition ($p_1$, $T_1$) **caractérisée par** un paramètre ($p$, $T$), par exemple une première température, une deuxième propriété de matériau ($M_2$, $\lambda$) étant détectée dans le procédé et la première propriété de matériau étant déterminée au moyen d'une corrélation ($M_1(M_2)$, $\rho(\lambda)$) entre la première propriété de matériau et la deuxième propriété de matériau, **caractérisé en ce que**, dans ladite corrélation ($M_1(M_2)$, $\rho(\lambda)$), on corrèle respectivement des valeurs de la première propriété de matériau ($M_1$, $\rho$), données dans la première condition ($p_1$, $T_1$), avec des valeurs de la deuxième propriété de matériau ($M_2$, $\lambda$), observées dans une deuxième condition modifiée ($p_3$, $T_3$), par exemple une deuxième température, les valeurs de la deuxième propriété de matériau dans la condition modifiée étant obtenues par une transformation, et la transformation résultant de la dépendance de la deuxième propriété de matériau par rapport au paramètre ($p$, $T$) caractérisant les conditions.

**2.** Procédé selon la revendication 1, dans lequel le paramètre caractérisant les conditions est la température et/ou la pression.

**3.** Procédé selon la revendication 1 ou 2, dans lequel la transformation est donnée par une relation linéaire, une expression polynomiale, une série de puissances ou une relation fonctionnelle.

**4.** Procédé selon l'une des revendications 1 à 3, dans lequel les valeurs de la deuxième propriété de matériau dans la condition modifiée sont obtenues par une transformation des valeurs détectées dans la première condition ($p_1$, $T_1$).

**5.** Procédé selon l'une des revendications 1 à 4, **caractérisé en ce que** la transformation des valeurs de la deuxième propriété de matériau ($M_2$, $\lambda$) est établie au moyen d'au moins un point de mesure détecté dans la première condition ($p_1$, $T_1$).

**6.** Procédé selon l'une des revendications 1 à 5, **caractérisé en ce que** la deuxième propriété de matériau est la conductivité thermique ($\lambda$) d'un mélange gazeux.

**7.** Procédé selon l'une des revendications 1 à 6, **caractérisé en ce que** la première propriété de matériau est la densité ($\rho$), la capacité thermique ($c_p$), la diffusivité thermique (conductivité thermique divisée par la densité et la capacité thermique), l'indice de méthane, l'indice de Wobbe, le pouvoir calorifique ou le besoin en air d'un mélange gazeux.

**8.** Procédé selon la revendication 7, dans lequel la diffusivité thermique est utilisée pour distinguer les gaz H /L.

**9.** Capteur pour déterminer une grandeur d'un mélange gazeux pertinente pour la technique de combustion, avec une unité d'évaluation qui est conçue pour exécuter un procédé selon l'une des revendications 1 à 8.

**10.** Capteur selon la revendication 9, comprenant en outre un bloc capteur (3), en particulier un bloc capteur (3) qui est regroupé avec l'unité d'évaluation dans une unité de construction commune.

**11.** Capteur selon la revendication 10, dans lequel le bloc capteur (3) est équipé d'un anémomètre à fil chaud, en particulier d'un anémomètre à fil chaud CMOS intégré, qui est exposé au mélange gazeux et au moyen duquel la dépendance de la conductivité thermique ($\lambda$) par rapport à la température peut être déterminée par mesure à au moins deux températures, par exemple en faisant varier la puissance de chauffage et/ou la présence de deux points de mesure de température à des distances de l'élément chauffant différentes du point de vue de la technique de conduction de chaleur.

12. Agencement de détection comprenant un capteur selon la revendication 10 ou 11, dans lequel le bloc capteur (3) est raccordé, au niveau de son entrée de gaz, à une conduite de mesure (2) menant à une conduite principale de gaz (1) et relié, au niveau de sa sortie de gaz, à une vanne d'arrêt (4) qui peut être fermée pendant la mesure et ouverte pour rincer le capteur.

13. Agencement de détection comprenant un capteur selon la revendication 10 ou 11, dans lequel le bloc capteur (3) est monté dans la conduite principale de gaz (1).

14. Agencement de détection comprenant un capteur selon la revendication 10 ou 11, dans lequel le bloc capteur (3) est raccordé, au niveau de son entrée de gaz et de sa sortie de gaz, à une conduite de mesure (2) (dérivation) menant à une conduite principale de gaz (1).

$M2(\varphi_B)$ - - - - - - - - - - - 8 - - - - - -> $M1(M2)$

5

$M2(\varphi'_B)=M2'$ ————————— 6 ————————> $M1(M2')$

7

## Fig. 1

**Fig. 2**

**Fig. 3**

**Fig. 4**

Fig. 5a

Fig. 5b

Fig. 5c

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7188519 B2
- EP 0554095 A2
- EP 0439950 A1
- EP 2015056 B1

**In der Beschreibung aufgeführte Nicht-Patentliteratur**

- **C. RAHMOUNI ; O. LE CORRE ; M TAZEROUT.** On-line determination of natural gas properties. *C.R: Mécanique,* 2003, vol. 331, 545-550
- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146